(19) Europäisches Patentamt
European Patent Office
Office européen des brevets

(11) Publication number: 0 138 575 B1

(12) **EUROPEAN PATENT SPECIFICATION**

(45) Date of publication of patent specification: **08.08.90**

(51) Int. Cl.$^5$: **C 07 C 69/157,** C 07 C 213/04

(21) Application number: **84306924.6**

(22) Date of filing: **10.10.84**

# (54) Process for the preparation of 2,3-cis-1,2,3,4-tetrahydro-5-(2-hydroxy-3-tert.butylamino)propoxyr-2,3-naphthalenediol and new intermediate for use therein.

(30) Priority: **11.10.83 FI 833685**

(43) Date of publication of application:
**24.04.85 Bulletin 85/17**

(45) Publication of the grant of the patent:
**08.08.90 Bulletin 90/32**

(84) Designated Contracting States:
**DE FR GB IT SE**

(56) References cited:
**DD-A- 129 322**
**DE-A-2 258 995**
**DE-A-2 332 706**

**TETRAHEDRON LETTERS, no. 23, pages 1973-1976, Pergamon Press, Oxford, GB; V. VANRHEENEN et al.: "An improved catalytic OsO4 oxidation of olefins to cis-1,2-glycols using tertiary amine oxides as the oxidant"**

(73) Proprietor: **Farmos-Yhtyma Oy**
**P.O. Box 425**
**SF-20101 Turku 10 (FI)**

(72) Inventor: **Ros, Hanspeter**
**Korivaara**
**SF-91500 Muhos (FI)**

(74) Representative: **Collier, Jeremy Austin Grey et al**
**J.A.Kemp & Co. 14, South Square Gray's Inn**
**London WC1R 5EU (GB)**

**EP 0 138 575 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to the preparation of 2,3-cis-1,2,3,4-tetrahydro-5-[(2-hydroxy-3-tert.butylamino) propoxy]-2,3-naphthalenediol, known as nadolol, which has the formula:

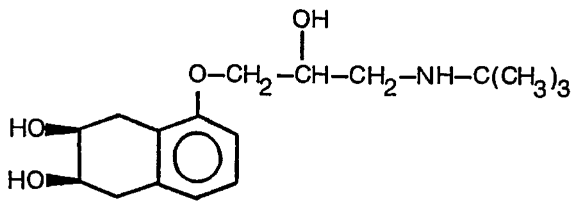

(I)

Nadolol is is a therapeutically valuable compound which has β-adrenergic blocking properties and is thus useful in the treatment of hypertension.

Nadolol has previously been prepared according to e.g., US-patent 3935267 and the DE-patents 2258995, 2332706 and 2333846. The known processes use, as starting material, cis-6,7-dihydroxy-5,6,7,8-tetrahydrol-1-naphthol of formula (III) below, which in the following is called cis-triol, which is prepared from 5,8-dihydro-1-naphthol-1-acetate of formula (II) below. For example in the DE-patent 2258995, a process is described which may be represented by the following reaction scheme:

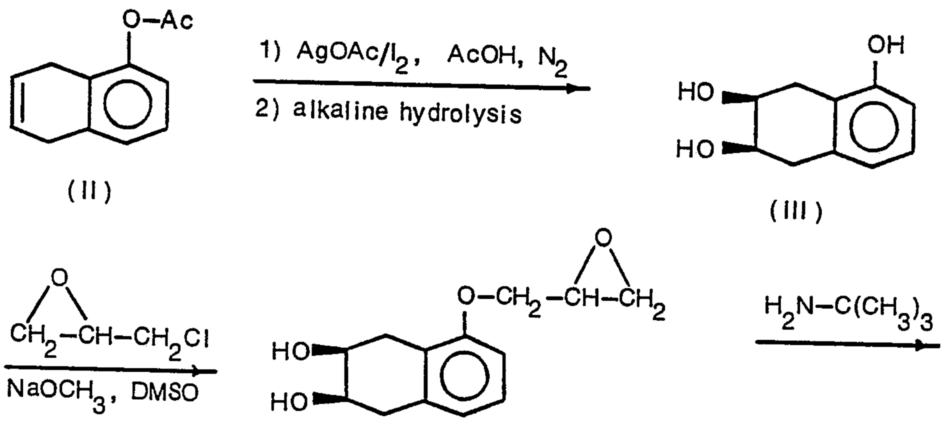


(II) (III) (I)

The end product is a mixture of the enantiomer pairs of two diastereoisomers.

In the DE patent 2332706 a process is described which may be represented as follows:

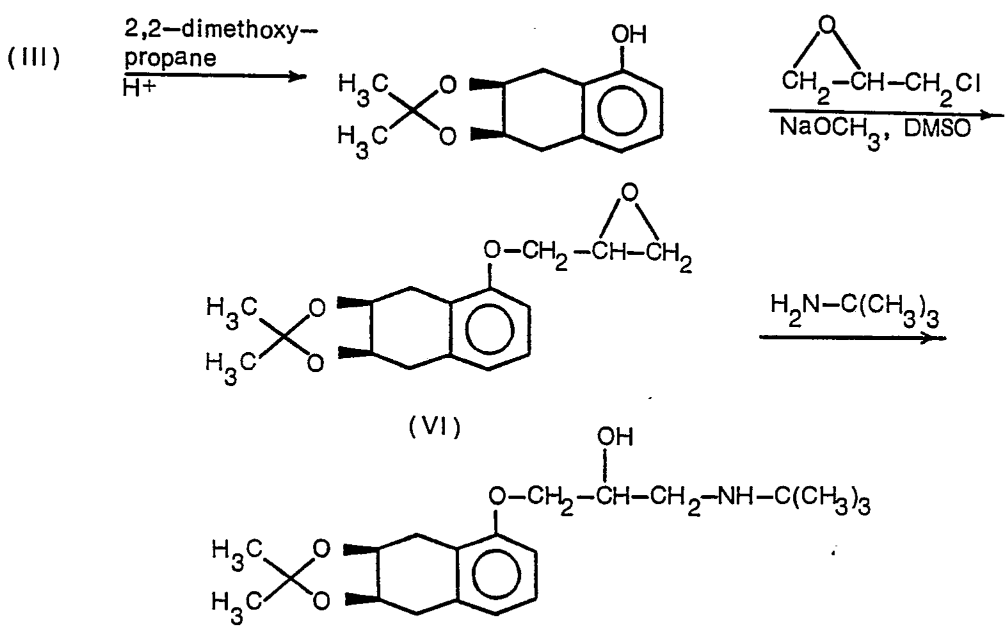


(III) (VI) (VII)

The ketal (VII) is subsequently converted into nadolol.

Both these processes involve the use of the cis-triol (III) which is toxic and therefore difficult to handle. Moreover, its preparation using silver acetate is expensive. Furthermore the yield, especially in the epoxidation step, is poor and the total yield II→I is only about 10%.

In the process of the present invention the difficulties inherent in the use of the cis-triol (III) are avoided by using cis-2,3-dihydroxy-1,2,3,4-tetrahydronaphthalene-5-acetate, of formula (IV) below, as an intermediate. Compound (IV), which is a new compound, is less toxic than the cis-triol (III), as it has no free phenolic hydroxyl, and it is also easier to purify and can be obtained in good yield. According to the present invention, this new intermediate is prepared by oxidizing 5,8-dihydro-1-naphthol-1-acetate with N-methylmorpholine-N-oxide in the presence of a catalytic amount of osmium tetroxide. This process may be represented thus:

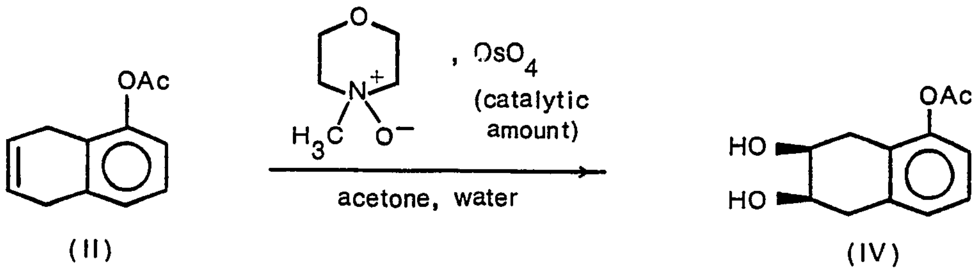


(II) (IV)

The oxidation of olefins to cis-1,2-glycols using N-methyl-morpholine-N-oxide and osmium tetroxide is known from the publication of V. Van Rheenan *et al.*, Tetrahedron Letters *17* (1976) 1973. However, in this publication the amount of $OsO_4$ used is 30 times greater than in the process of the present invention and the olefins in question are different. In the present process osmium tetroxide is added successively to the reaction until the total amount which has been added is $6.7 \times 10^{-5}$ to $3.3 \times 10^{-4}$ moles per mole of 5,8-dihydro-1-naphthol-1-acetate.

The monoacetate (IV) may then be converted into nadolol according to the following synthesis scheme:

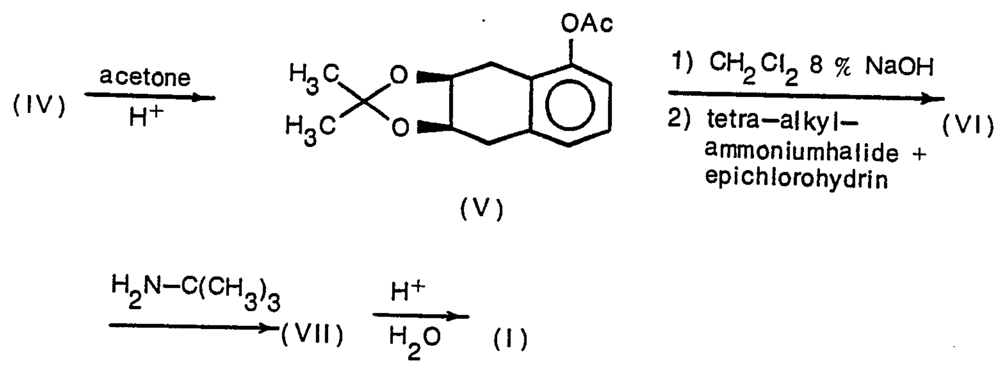


In place of the usual O-alkylation, a phase transfer technique is used in the conversion of compound (IV) into the desired end product, which in this way is obtained in considerably higher yield and whose isolation becomes easier.

The present invention thus provides a process for the preparation of 2,3-cis-1,2,3,4-tetrahydro-5-[(2-hydroxy-3-tert.butylamino) propoxy]-2,3-naphthalenediol of formula:

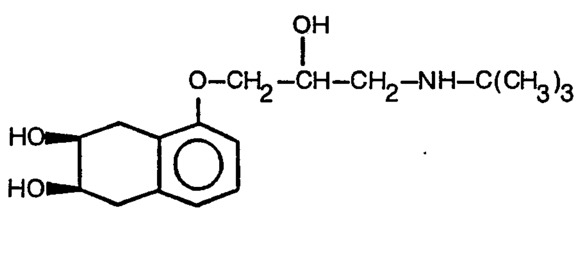

(I)

which comprises oxidizing 5,8-dihydro-1-naphthol-1-acetate of formula:

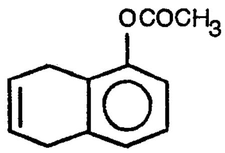

(II)

with N-methylmorpholine-N-oxide in the presence of a catalytic amount of osmium tetroxide to give cis-2,3-dihydroxy-1,2,3,4-tetrahydronaphthalene-5-acetate of formula:

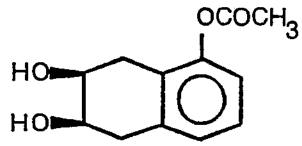

(IV)

reacting the latter with acetone to give the acetonide of formula

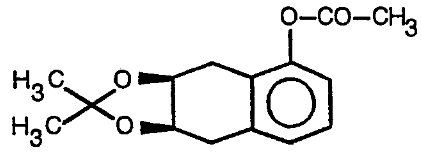

(V)

reacting the said acetonide with a base until the acetate group is hydrolysed and then with epichlorohydrin in the presence of a base and a quarternary alkylammoniumhalide catalyst to give the compound of formula

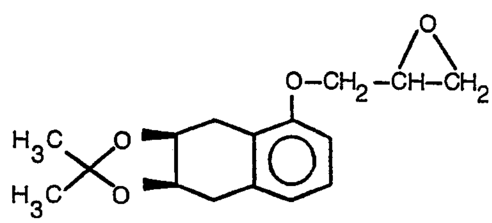

(VI)

which is then reacted with tert.butylamine to give 2,3-cis-1,2,3,4-tetrahydro-5-[(2-hydroxy-3-tert-butylamino) propoxy]-2,3-naphthalenediol acetonide, which is hydrolysed to provide the compound of formula (I).

As compared to known processes, the process of the present invention has the following advantages:

1. The starting material of formula (IV) is considerably cheaper than the cis-triol (III). Silver acetate is a precious metal salt and its price fluctuations are considerable and unpredictable. The actual cost of manufacturing the cis-triol (III) is therefore difficult to estimate. Moreover, although osmium tetroxide is not a cheap reagent the weight of silver acetate used is 20000 times greater than the amount of osmium tetroxide used in the present invention.

2. Since the monoacetate (IV) is a protected phenol it is not prone to self-oxidation. Because of this it is both more stable and easier to purify than the cis-triol (III). In addition, it is less toxic.

3. The phase transfer is, when considering the whole synthesis, much more advantageous than ordinary O-alkylation. According to DE-patent 2258995 solvents are used which are difficult to remove by distillation. In consequence the product of each step has to be isolated by crystallization. By using the phase transfer technique, all solvents in the epoxidation step are removed by distillation or by separation of phases. Therefore the step (IV)→(I) can be performed in the same reaction vessel without isolation of any intermediate. This makes the process very advantageous on a technical scale.

4. Because the O-alkylation of the phenolate ion with epichlorohydrin under phase transfer conditions is much faster than under ordinary conditions, the reaction can be performed at lower temperatures, which gives the epoxy compound (VI) in higher purity.

5. The solvents used in the phase transfer are cheaper and can easily be recycled.

6. The overall yield of the process is higher (35—40%).

The solvent used in the preparation of the cis-triol monoacetate is a mixture of an organic solvent and water, preferably an acetone-water mixture. Catalytic amounts of $OsO_4$ dissolved in, e.g., tert.butanol are added. The reaction temperature is about 20—30°C.

The cis-triol monoacetate acetonide (V) may be prepared in acetone in the presence of *p*-toluenesulfonic acid at room temperature. The acetate group is hydrolysed in diluted, e.g. 8%, NaOH-solution at about 60—70°C. The cis-triol acetonide obtained is not isolated, but is reacted in the above-mentioned NaOH-solution with epichlorohydrin using the phase transfer-technique. The organic phase used may be a chlorinated hydrocarbon such as dichloromethane, and the catalyst maybe a quaternary alkylammonium halide, preferably tetrabutylammonium bromide. The temperature is preferably about 40°C.

The reaction of the product (VI) with tert. butylamine preferably takes place in an organic solvent boiling below 100°C, such as an alcohol, preferably methanol, a cyclic or aliphatic ether, or a chlorinated hydrocarbon.

Finally, the acetonide group in the compound (VII) is hydrolysed, e.g. in $CH_2Cl_2$-water mixture, using an acidic aqueous phase (pH about 1), preferably at room temperature.

The following Examples illustrate the invention.

Example 1

Cis-2,3-dihydroxy-1,2,3,4-tetrahydronaphthalene-5-acetate (IV).

10 g. (53 mmol) of 5,8-dihydro-1-naphthol-1-acetate (II) is placed in a round-bottomed flask and 10o0 ml acetone, 13.7 g N-methylmorpholine-N-oxide and 14 ml distilled water are added. Then is added 13 µl 25% $OSO_4$ in tert.butanol solution and the mixture is stirred at 23—30°C for 24 h. Then is added again 5 µl of the above mentioned $OsO_4$-solution and the mixture is stirred for 24 h at 23°C. This treatment is repeated 6 more times. All the starting material has then reacted. 1 g sodium bisulfite is added and the mixture is stirred for one hour. The solution is filtered and the acetone distilled off under vacuum at a temperature below 30°C. The product crystallizes at the end of the distillation. About 10 ml toluene are added and the mixture is cooled to 5°C and stirred at this temperature for one hour. The product is filtered off and washed with toluene and water. The dried product weighs 8.8 ag (75%), m.p. 141—143°C.

Example 2

2,3-cis-1,2,3,4-tetrahydro-5-[(2-hydroxy-3-tert.butylamino)-propoxy]-2,3-naphthalenediol (nadolol) (I).

9.5 g (42.6 mmol) cis-2,3-dihydroxy-1,2,3,4-tetrahydronaphthalene-5-acetate is placed in a round-bottomed flask and 60 ml acetone and 0.5 g p-toluenesulfonic acid are added. The reaction mixture is stirred for 2 hours at room temperature. The acetone is distilled off and 60 ml 8% sodium hydroxide solution are added. The mixture is heated to 68—70°C until the acetate group is hydrolysed. The hydrolysis is followed by thinlayer chromatography (using $CH_2Cl_2$:$CH_3OH$ = 95:5). When the reaction is completed, the mixture is cooled and 40 ml dichloromethane, 0.5 g tetrabutylammonium bromide, and 11.8 g epichlorohdrin dissolved in 12 ml dichloromethane are added. The mixture is stirred at about 40°C and the formation of epoxide is followed by thinlayer chromatography (using toluene: ethyl acetate: chloroform: acetone = 85: 15: 5: 5). When the reaction is complete, the mixture is cooled to room temperature and the layers separated. Dichloromethane and unreacted epichlorohydrin are distilled off. 20 ml methanol and 20 ml tert.butylamine are added to the distillation residue and the mixture heated for 3 hours at 60°C. The solvents are removed by distillation in vacuum and 40 ml dichloromethane and 40 ml water are added. The pH of the aqueous layer is adjusted to 0—1 and the acidic mixture stirred at room temperature 2—3 hours. The hydrolysis of the acetonide can be followed by thinlayer chromatography (using toluene: ethyl acetate: chloroform: ethanol: conc. $NH_3$ solution = 80: 30: 10: 75: 15). The pH of the aqueous phase is then adjusted to about 12 and the aqueous phase is extracted three times with dichloromethane. The extract is treated with activated carbon and the dichloromethane is evaporated to obtain the desired product, 2,3-cis-1,2,3,4-tetrahydro-5-[(2-hydroxy-3-(tert.butylamino)-propoxy]-2,3-naphthalenediol (I) 4.6 g, as a colourless powder, m.p. 126—136°C. The overall yield is 35—40%.

**Claims**

1. A process for the preparation of 2,3-cis-1,2,3,4-tetrahydro-5-[(2-hydroxy-3-tert.butylamino) propoxy]-2,3-naphthalenediol of formula:

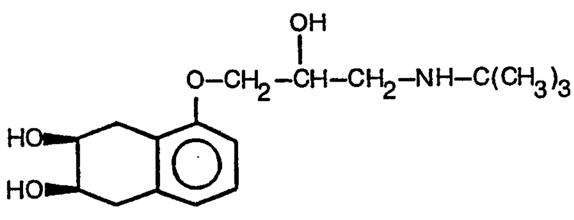

(I)

which comprises oxidizing 5,8-dihydro-1-naphthol-1-acetate of formula:

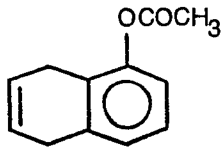

(II)

with N-methylmorpholine-N-oxide in the presence of osmium tetroxide which is added successively to the reaction until the total amount of osmium tetroxide which has been added is $6.7 \times 10^{-5}$ to $3.3 \times 10^{-4}$ moles per mole of 5,8-dihydro-1-naphthol-1-acetate, to give cis-2,3-dihydroxy-1,2,3,4-tetrahydronaphthalene-5-acetate of formula:

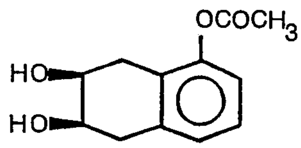

(IV)

reacting the latter with acetone to give the acetonide of formula:

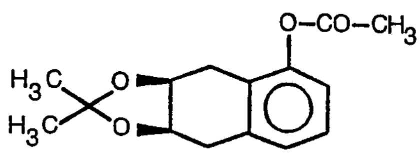

(V)

reacting the aid acetonide with a base until the acetate group is hydrolysed and then with epichlorohydrin in the presence of a base and a quaternary alkylammoniumhalide catalyst to give the compound of formula

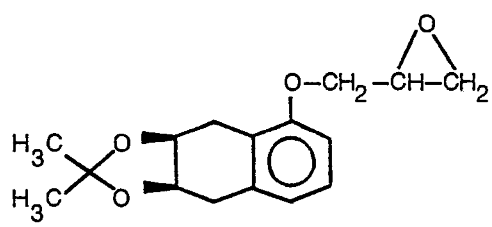

(VI)

which is then reacted with tert.butylamine to give 2,3-cis-1,2,3,4-tertrahydro-5-[(2-hydroxy-3-tert-butylamino) propoxy]-2,3-naphthalenediol acetonide, which is hydrolysed to provide the compound of formula (I).

2. A process according to claim 1 wherein the said oxidation with N-methylmorpholine-N-oxide is effected in an aqueous organic solvent.

3. A process according to claim 1 or 2 wherein the acetonide is reacted with an aqueous solution of an alkali metal hydroxide and then with epichlorohydrin in the presence of a water-immiscible organic solvent and the quaternary ammonium halide catalyst.

4. A process according to claim 3 wherein the acetonide is reacted with an 8% sodium hydroxide solution and then with epichlorohydrin in the presence of dichloromethane using, as catalyst, tetrabutylammonium bromide.

5. A process according to any one of claims 1 to 4 wherein the compound of formula (VI) is reacted with tert-butylamine in an organic solvent boiling below 100°C.

6. A process according to any one of claims 1 to 5 wherein the 2,3-cis-1,2,3,4-tetrahydro-5-[(2-hydroxy-3-tert.butylamino)propoxy]-2,3-naphthalenediol acetonide is hydrolysed in acid dichloromethane-water solution.

7. Process in the preparation of cis-2,3-dihydroxy-1,2,3,4-tetrahydronaphthalene-5-acetate which comprises oxidizing 5,8-dihydro-1-naphthol-1-acetate of formula:

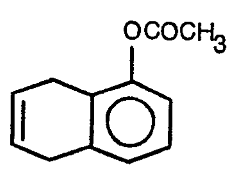

(II)

with N-methylmorpholine-N-oxide in the presence of osmium tetroxide which is added successively to the reaction until the total amount of osmium tetroxide which has been added is $6.7 \times 10^{-5}$ o $3.3 \times 10^{-4}$ moles per mole of 5,8-dihydro-1-naphthol-1-acetate.

**Patentansprüche**

1. Verfahren zur Herstellung von 2,3-dis-1,2,3,4-Tetrahydro-5-[(2-Hydroxy-3-tert-Butylamino)propoxy]-2,3-Naphthalindiol de allgemeinen Formel:

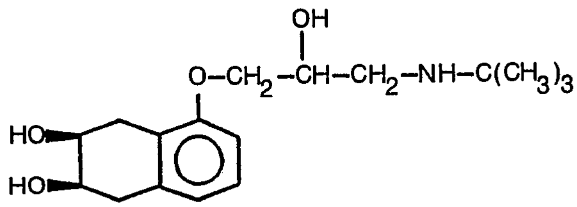

(I)

bei welchem 5,8-Dihydro-1-Naphthol-1-Acetat der allgemeinen Formel:

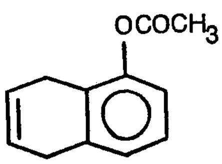

(II)

mit Methylmorpholin-N-Oxid in Gegenwart von Osmiumtetroxid oxidiert wird, das nach und nach der Reaktion zugesetzt wird, bis die zugesetzte Gesamtmenge an Osmiumtetroxid $6{,}7 \times 10^{-5}$ bis $3{,}3 \times 10^{-4}$ Mol pro Mol 5,8-Dihydro-1-Naphthol-1-Acetat beträgt, worauf man cis-2,3-Dihydroxy-1,2,3,4-Tetrahydronaphthalin-5-Acetat der allgemeinen Formel:

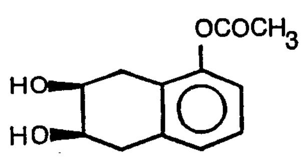

(IV)

erhält, bei welchem das so erhaltene Produkt mit Aceton umgesetzt wird, worauf man das Acetonid der allgemeinen Formel:

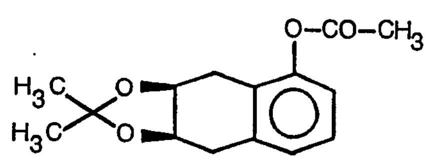

(V)

erhält, das mit einer Base bis zur Hydrolisierung der Acetatgruppe umgesetzt und anschließend in Gegenwart einer Base und eines quaternären alkylammoniumhalogenidhaltigen Katalysators mit Epichlorhydrin zu einer Verbindung der allgemeinen Formel:

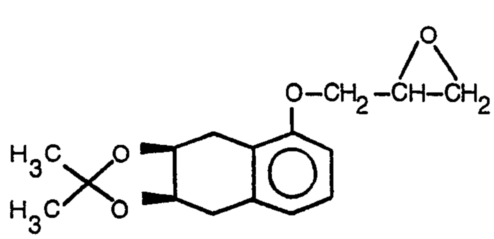

(VI)

umgesetzt wird, die anschließend mit tert-Butylamin zu 2,3-cis-1,2,3,4-Tetrahydro-5-[(2-Hydroxy-3-tert-Butylamino)propoxy]-2,3-Naphthalindiolacetonid umgesetzt wird, das nach Hydrolyse eine Verbindung der allgemeinen Formel (I) ergibt.

2. Verfahren nach Anspruch 1, bei welchem die Oxidierung mit N-Methylmorpholin-N-oxid in einem wäßrigen organischen Lösungsmittel durchgeführt wird.

3. Verfahren nach Anspruch 1 oder 2, bei welchem das Acetonid mit eine wäßrigenn Lösung eines Alkalimetallhydroxids und anschließend mit Epichlorhydrin in Gegenwart eines mit Wasser unvermischbaren organischen Lösungsmittels und des quarternären ammoniumhalogenidhaltigen Katalysators umgesetzt wird.

4. Verfahren nach Anspruch 3, bei welchem das Acetonid mit einer 8%igen Hydroxidlösung und anschließend mit Epichlorhydrin in Gegenwart von Dichlormethan unter Einsatz von Tetrabutylammoniumbromid als Katalysator umgesetzt wird.

5. Verfahren nach einem der Ansprüche 1 bis 4, bei welchem die Verbindung der allgemeinen Formel (VI) mit tert-Butylamin in einem organischen Lösungsmittel umgesetzt wird, dessen Siedepunkt unter 100°C liegt.

6. Verfahren nach einem der Ansprüche 1 bis 5, bei welchem das 2,3-bis-1,2,3,4-Tetrahydro-5-[(2-Hydroxy-3-tert-Butylamino)propoxy]-2,3-Naphthalindiolacetonid einer Hydrolyse in einer sauren Dichlormethan-Wasser-Lösung unterzogen wird.

7. Verfahren zur Herstellung von cis-2,3-Dihydroxy-1,2,3,4-Tetrahydronaphthalin-5-acetat, bei welchem 5,8-Dihydro-1-Naphthol-1-acetat der allgemeinen Formel:

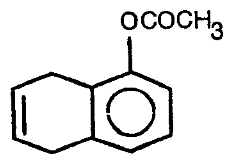

(II)

mit N-Methylmorpholin-N-oxid in Gegenwart von Osmiumatetroxid oxidiert wird, das nach und nach der Reaktion zugegeben wird, bis die Gesamtmenge an zugesetztem Osmiumtetroxid $6,7 \times 10^{-5}$ bis $3,3 \times 10^{-4}$ Mol pro Mol 5,8-Dihydro-1-Naphthol-1-acetat beträgt.

**Revendications**

1. Procédé de préparation du 2,3-cis-1,2,3,4-tétrahydro-5-[(2-hydroxy-3-tert-butylamino) propoxy]-2,3-naphtalènediol de formule:

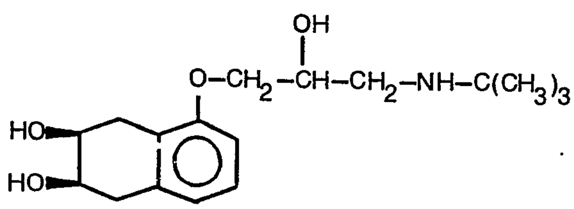

(I)

comprenant l'oxydation du 5,8-dihydro-1-naphtol-1-acétate de formule:

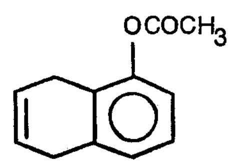

(II)

par le N-méthylmorpholine-N-oxyde en présence de tétroxyde d'osmium qui est ajouté progressivement à la réaction jusqu'à ce que la quantité totale de tétroxyde d'osmium ajoutée soit comprise entre $6,7 \times 10^{-5}$ et $3,3 \times 10^{-4}$ moles par mole de 5,8-dihydro-1-naphtol-1-acétate, pour obtenir le cis-2,3-dihydroxy-1,2,3,4-tétrahydronaphtalène-5-acétate de formule:

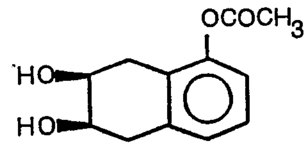

(IV)

puis la réaction de ce dernier avec l'acétone pour obtenir le dérivé acétonique de formule:

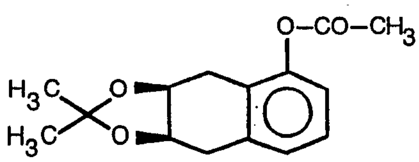

(V)

puis la réaction dudit dérivé acétonique avec une base jusqu'à ce que le groupe acétate soit hydroylsé et ensuite avec l'épichlorhydrine en présence d'une base et d'un catalyseur halogénure d'alkylammonium quaternaire pour obtenir le composé de formule:

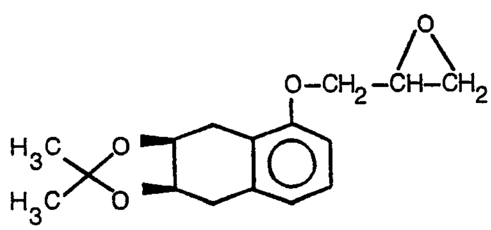

(VI)

qui est mis à réagir ensuite avec la tert-butylamine pour donner le dérivé acétonique du 2,3-cis-1,2,3,4-tétrahydro-5-[(2-hydroxy-3-tert-butylamino)propoxy]-2,3-naphtalènediol qui est hydrolysé pour produire le composé de formule I.

2. Procédé selon la revendication 1 dans lequel ladite oxydation par le N-méthylmorpholine-N-oxyde est réalisée dans un solvant hydro-organique.

3. Procédé selon la revendication 1 ou 2 dans lequel le dérivé acétonique est mis à réagir avec une solution aqueuse d'un hydroxyde de métal alcalin puis avec l'épichlorhydrine en présence d'un solvant organic non miscible à l'eau et d'un catalyseur halogénure d'ammonium quaternaire.

4. Procédé selon la revendication 3 dans lequel le dérivé acétonique est mis à réagir avec une solution d'hydroxyde de sodium à 8% puis avec l'épichlorhydrine en présence de dichlorméthane et, comme catalyseur, de bromure de tétrabutylammonium.

5. Procédé selon l'une quelconque des revendications 1 à 4 dans lequel le composé de formule (VI) est mis à réagir avec la tert-butylamine dans un solvant organique de point d'ébullition inférieur à 100°C.

6. Procédé selon l'une quelconque des revendications 1 à 5 dans lequel le dérivé acétonique du 2,3-dix-1,2,3,4-tétrahydro-5-[(2-hydroxy-3-tert.butylamino) propoxy]-2,3-naphtalènediol est hydrolysé en solution acide dichlorométhane-eau.

7. Procédé de préparation du cis-2,3-dihydroxy-1,2,3,4-tétrahydro-naphtalène-5-acétate comprenant l'oxydation du 5,8-dihydro-1-naphtol-1-acétate de formule

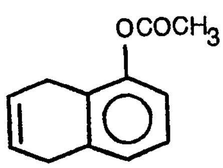

(II)

par le N-méthylmorpholine-N-oxyde en présence de tétroxyde d'osmium qui est ajouté progressivement à la réaction jusqu'à ce que la quantité totale de tétroxyde d'osmium ajoutée soit comprise entre $6,7 \times 10^{-5}$ et $3,3 \times 10^{-4}$ moles par mole de 5,8-dihydro-1-naphtol-1-acétate.